# EUROPÄISCHE PATENTSCHRIFT

(11) **EP 1 113 816 B1**
(45) Veröffentlichungstag und Bekanntmachung des Hinweises auf die Patenterteilung: **11.01.2006**
(21) Anmeldenummer: 99944154.6
(22) Anmeldetag: 14.09.1999
(51) Int. Cl.: A61K 39/145, A61K 39/39

(54) **INFLUENZAVIRUS-IMPFSTOFFZUSAMMENSETZUNG**
INFLUENZA VIRUS VACCINE COMPOSITION
COMPOSITION VACCINALE CONTRE LE VIRUS DE LA GRIPPE

(30) Priorität: 15.09.1998 AT 155598
(43) Veröffentlichungstag der Anmeldung: 11.07.2001
(73) Patentinhaber: Baxter Healthcare S.A., 8304 Wallisellen (CH)
(72) Erfinder: KISTNER, Otfried, A-1040 Wien (AT); BARRETT, Noel, A-3400 Klosterneuburg/Weidling (AT); MUNDT, Wolfgang, A-1080 Wien (AT); DORNER, Friedrich, A-1230 Wien (AT)
(74) Vertreter: Perrey, Ralf
(86) Internationale Anmeldenummer: PCT/AT1999/000223
(87) Internationale Veröffentlichungsnummer: WO 2000/015251

(56) Entgegenhaltungen:
- WO-A-96/15231
- KISTNER O ET AL: "Development of a mammalian cell (Vero) derived candidate influenza virus vaccine." VACCINE, (1998 MAY-JUN) 16 (9-10) 960-8. , XP004122292
- GUARNACCIA S ET AL: "A comparative immunogenicity-reactogenicity dose-response study of influenza vaccine." ANNALS OF ALLERGY, (1990 SEP) 65 (3) 218-21. , XP000887044 in der Anmeldung erwähnt
- MERTEN O W ET AL: "Production of influenza virus in serum-free mammalian cell cultures." DEVELOPMENTS IN BIOLOGICAL STANDARDIZATION, (1999) 98 23-37;DISCUSSION 73-4. , XP000909398
- KISTNER O ET AL: "Development of a Vero cell-derived influenza whole virus vaccine." DEVELOPMENTS IN BIOLOGICAL STANDARDIZATION, (1999) 98 101-10;DISCUSSION 111. , XP000909542

## Beschreibung

Die Erfindung betrifft eine Influenzavirus-Impfstoffzusammensetzung mit einem reduzierten HA-Antigengehalt und Aluminium als Adjuvans. Desweiteren betrifft die Erfindung die Verwendung der Impfstoffzusammensetzung zur Herstellung eines Arzneimittels und zur Induktion einer effektiven Immunantwort in höheren Wirbeltieren, insbesondere beim Menschen.

Influenza-Virusinfektionen stellen ein immer höheres gesundheitliches Risiko insbesondere bei älteren Menschen und bei Personen mit chronischen Krankheiten dar, da die Infektion bei diesen Personengruppen oftmals zu einer erhöhten Mortalitätsrate führt. Seit der Einführung einer inaktivierten Influenza-Vakzine, enthaltend inaktiviertes Virusmaterial aus infizierten bebrüteten Hühnereiern, in den 40iger Jahren ist das Risiko und der Verlauf der Infektion, sowie die Mortalitätsrate bei älteren Menschen gesunken.

Die Gesundheitsbehörden empfehlen für eine effektive Vakzine, die ein positives Verhältnis zwischen Vakzindosis und IgG-Antikörperantwort ergibt, eine Vakzindosis zwischen 10 µg bis 15 µg HA (Hämagglutination)-Antigen pro Dosis.

Die kurzfristige Herstellung von großen Antigenmengen zur Vakzinproduktion bei einer Pandemie, insbesondere mit der bisher eingesetzten Methode über bebrütete Hühnereier, ist nicht nur sehr arbeitsintensiv und erfordert die Bereitstellung großer Mengen an Eiern, sondern erfordert aufgrund der kurzen Zeitintervalle von der Identifizierung des Virustyps bis zur Bereitstellung des Impfstoffs einen großen logistischen Aufwand. Zudem wird es in Zukunft durch ein erhöhtes Bewußtsein, insbesondere Risikogruppe rechtzeitig zu impfen, zu einer erhöhten Nachfrage für eine effektive Vakzine kommen.

Die derzeit geschätzte Effizienz einer humanen Influenzavirus-Vakzine liegt im Bereich zwischen 30% und 80%. Um die Effizienz zu erhöhen, wurde vorgeschlagen, die Impfstoffdosis zu erhöhen. Untersuchungen von Palache et al. (1993, Vaccine 11:3-9) und Palache et al. (1993, Vaccine 11:892-908) zeigen jedoch, daß die Erhöhung der Vakzindosis nicht immer eine adäquate Methode ist, die Antikörperantwort und die Protektivität zu erhöhen, da das Ausmaß der Immunantwort sehr stark vom Antigen abhängt. Es wurde zwar gefunden, daß eine Tendenz zu einer erhöhten Immunantwort besteht, wenn eine höhere Antigendosis eingesetzt wird, die jedoch oberhalb eines Bereiches von 10 µg bis 15 µg weniger ausgeprägt ist, und die durch die hohe Impfstoffdosis verursachten Nebenwirkungen oftmals nicht rechtfertigt.

Andere Ansätze zur Erhöhung der Immunantwort, insbesondere bei älteren Menschen, galten dem Einsatz von zusätzlichen Adjuvantien. Antigenpräparationen enthaltend Mineralöl-Emulsionen zeigten dabei zwar eine verbesserte Immunantwort gegenüber nicht-adjuvantierten Vakzinen, aber auch erhöhte Nebenwirkungen (Fukumi et al., 1967, In: Symposium Series. Immunobiology Standard, Vol. 6: 237 ff., Karger, Basel, New York). Das einzige für die Anwendung am Menschen zugelassene Adjuvans, Aluminium, zeigte in klinischen Studien am Menschen keine Verstärkung der Immunogenität von Influenzavirusantigen, obwohl die Immunantwort bei Mäusen durch das Adjuvans verstärkt war (Davenport et al., 1968, J. Immunol. 100: 1139-1140, Nicholson et al., 1979, J. Biol. Stand. 7:123-136, Bachmayr et al., 1976, Split and subunit vacccines. In: Influenza: Virus, Vaccines, Strategy (Ed. Selby, P.) Academic Press, New York, pp. 149-152, Jennings et al., 1981, J. Hyg. 81:1-16). Untersuchungen von Skea et al. (1993, Vaccine 11:1018-1026) zur Erhöhung der Immunantwort gegenüber einer Influenzavirus-Vakzine zeigten ebenfalls, daß Aluminiumverbindungen alleine schwache Adjuvantien für Influenzavirus-Antigen sind. Skea et al. *supra* konnten jedoch durch Erhöhung der Adhäsion von HA-Antigen an Aluminium durch spezifische anti-Influenzavirus-HA-Antikörper eine 1500-fach höhere Immunogenität in Mäusen und eine um 5-fach erhöhte Immunogenität in Kaninchen im Vergleich zu Aluminium alleine erzielen. Sie folgerten daraus, daß die Adjuvansaktivität von Aluminium entscheidend durch die Verstärkung der physikalischen Adhäsion zwischen Antigen und Adjuvans erhöht werden kann. Daten für Versuche an höheren Säugern oder Menschen wurden jedoch nicht gezeigt.

Es wurde eine Reihe von Untersuchungen zur Austestung von Adjuvantien mit einer akzeptablen Reaktivität durchgeführt. So wurden immunstimulierende Komplexe (ISCOMS™), Öl-in-Wasser- Adjuvantien (Coulter et al., 1998, Vaccine 16: 1243-1253), Vaxcel™, TiterMax™, Syntex, AlPO₄, Freund's komplettes und inkomplettes Adjuvans (Robuccio et al., 1995, Lab. Animal Sci. 45:420-426), Poly(amidoamin)-Dendrimer (WO 97/28809) und MF59 (Keitel et al., 1993, Vaccine 11:909-913, Martin, 1997, Biologicals 25:209-213) auf ihre Adjuvans-Aktivität auf Influenzavirus-HA-Antigen getestet. Es zeigte sich, daß die eingesetzten Adjuvantien in unterschiedlichem Ausmaß die Immunantwort verstärken. Andererseits verursachen sie auch, abhängig von der eingesetzten Adjuvanskonzentration, mehr oder weniger heftige Nebenwirkungen.

Ein weiteres Problem bei der Vakzinierung insbesondere von Hochrisiko-Gruppen, wie Allergikern und Asthmapatienten mit konventionellem Influenzavirus-Impfstoff aus Hühnereiern besteht darin, daß diese Personen besonders häufig Nebenwirkungsreaktionen, wie allergische Reaktionen gegen Hühnereiweißproteine zeigten. Oftmals war auch eine geringe Menge an Hühnereiweiß ausreichend, um lebensbedrohliche, hypersensitive, allergische Reaktionen auszulösen. Es wurde daher zu einer allgemein üblichen Praxis, die Influenzavirus-Vakzine 1:10 zu verdünnen, um die mit dem Impfstoff verabreichte Menge an Hühnereiweiß-Proteinen zu reduzieren. Untersuchungen von Guarnaccia et al. (1990, Ann. Allergy 65:218-221) zeigen jedoch, daß die Herabsetzung der normal verabreichten Menge an Influenzavirus-Antigen auch zu einer starken Reduktion der Immunantwort führt, und empfehlen daher diese Praxis der Antigenmengenreduktion nicht durchzuführen, da damit kein ausreichender Schutz der Patienten gegen eine VirusInfektion gesichert ist.

Aufgabe der vorliegenden Erfindung ist es daher, eine Impfstoffzusammensetzung, die die oben beschriebenen Nachteile, wie hohe Antigendosis, Nebenwirkungen-auslösende Adjuvantien oder allergische Reaktionen gegen Hühnereiweiß-Proteine nicht aufweist, zur Verfügung zu stellen.

Die Aufgabe wird erfindungsgemäß gelöst durch Zurverfügungstellen einer Influenzavirus-Vakzine, enthaltend eine aus Zellkultur gewonnene Influenzavirusantige präparation, mit einem HA-Antigengehalt von maximal zwischen 1 µg und 5 µg HA-Antigen/Dosis und Aluminium als Adjuvans.

Es wurde analog zu den Ergebnissen von Guarnaccia et al. (1990, Ann. Allergy 65:218-221) gefunden, daß eine Reduktion der Influenza-Antigenmenge in einer Impfstoffdosis auch zu einer verminderten Immunantwort bei Mäusen führt. In Übereinstimmung mit den Ergebnissen von Davenport et al. (1968, J. Immunol. 100:1139-1140, Hjorth et al., 1997, Vaccine 15:541-546) konnte ebenfalls gezeigt werden, daß bei einer Impfstoffzusammensetzung, enthaltend über konventionelle Methoden aus infizierten bebrüteten Hühnereiern isoliertes Influenzavirusantigen bei gleichzeitiger Anwesenheit von Aluminium als Adjuvans, der HA-Titer verstärkt wird. Die Zugabe von Aluminium führte jedoch auch bei einer Impfstoffdosis mit einem stark reduzierten Antigengehalt auf 1/10 der normalerweise ausreichenden Antigenmenge (1,5 µg) zu einem gleich hohen HA-Titer wie der Impfstoff, enthaltend die 10-fache Antigenmenge (Tabelle 1).

Überraschenderweise wurde jedoch gefunden, daß die gleiche Menge eines Antigens des gleichen Influenzavirus-Stammes, isoliert aus einer mit Influenzavirus infizierten Zellkultur, einen 2-fach höheren Antikörpertiter induziert als Antigen, isoliert aus Hühnereiern. Zudem konnte gezeigt werden, daß die Zugabe von Aluminium in einer Präparation, enthaltend aus Zellkultur isoliertes Antigen, die Immunantwort des Antigens bei einer normal hohen Antigendosis nicht oder nur unwesentlich verstärkt, während bei Immunisierung mit einer Präparation, enthaltend eine weitaus geringere Dosis dieses Antigens und Aluminium als Adjuvans, der HA-Titer sogar über dem mit der höheren Antigendosis lag (Tabelle 1) und damit zu einer verstärkten Immunantwort führt.

Die Adjuvans-Aktivität von Aluminium ist daher bei Influenzavirusantigen, isoliert aus Zellkultur, wesentlich höher als bei einem Antigen, isoliert aus bebrüteten Eiern. Dies war insbesondere daher überraschend, da in der Fachwelt allgemein bekannt war, daß die Immunantwort bei einem Antigengehalt von ≤10 µg Antigen/Dosis stark reduziert ist (Guarnaccia et al., 1990, Ann.

Allergy 65:218-221) und daß Aluminium nur einen schwach adjuvantierenden Effekt auf Influenza-Antigen hat.

Zudem war noch unerwartet, daß insbesondere mit einer Influenzavirus-Antigenpräparation, hergestellt aus gereinigtem Antigen aus Zellkultur, und der Kombination von reduziertem Influenzavirus-Antigengehalt und Aluminium als Adjuvans, eine wesentlich höhere Immunantwort erreicht wird im Vergleich zu einer Präparation mit einem Gehalt von ≥ 10 µg Antigen/Dosis ohne Adjuvans. Die Immunogenität des aus Zellkultur isolierten Influenzavirusantigens konnte durch Reduktion des Antigengehalts im Impfstoff und durch Anwesenheit von Aluminium als Adjuvans um das 10-fache gesteigert werden.

Gemäß einem besonderen Aspekt der Erfindung enthält die erfindungsgemäße Influenzavirus-Vakzine einen Gehalt von 1 µg bis 2,5 µg HA-Antigen/Dosis. Besonders bevorzugt enthält die erfindungsgemäße Vakzinformulierung 1,5 µg HA-Antigen/Dosis.

Die erfindungsgemäße Influenzavirus-Vakzine enthält Aluminium vorzugsweise in Form von Aluminiumhydroxid (Al(OH)₃) oder Aluminiumphosphat (AlPO₄). Die Konzentration des Aluminiums kann dabei vorzugsweise mit einer Endkonzentration von 0,05% bis 0,5% in der Vakzinformulierung vorliegen.

Der besondere Vorteil der erfindungsgemäßen Vakzinformulierung liegt neben der erhöhten Immunogenität der Präparation auch darin, daß sie (i) durch den reduzierten HA-Antigengehalt und (ii) durch Verwendung eines Adjuvans, das über lange Jahre erprobt und seine Verwendung am Menschen zugelassen ist, fast vollständig nebenwirkungsfrei ist.

Zudem können aus einer normalerweise für eine Impfdosis notwendigen Antigenmenge bis zu 10-mal soviel Impfdosen hergestellt werden (15 µg/Dosis vs. 1,5 µg/Dosis). Dies reduziert nicht nur den für die Antigenproduktion notwendigen industriellen Aufwand, sondern löst auch die möglichen bei einer Pandemie auftretenden Probleme der schnellen Bereitstellung von mehreren Millionen Impfdosen.

Es konnte im Rahmen der vorliegenden Erfindung nicht nur gezeigt werden, daß die erfindungsgemäße Vakzine in Mäusen eine verbesserten Immunantwort induziert, sondern es wurde auch in Versuchen mit Schimpansen gezeigt, daß die erfindungsgemäße Vakzinpräparation auch bei höheren Säugern wirkt. Dies war insbesondere deshalb überraschend, da nicht zu erwarten war, daß (i) das Immunadsorbens Aluminium einen immunverstärkenden Effekt in höheren Säugern aufweist und (ii) daß die Reduktion des Antigengehalts eine wesentlich höhere Immunantwort in Schimpansen auslöst. Durch diese Versuche wurde ebenfalls gezeigt, daß die erfindungsgemäße Präparation fast völlig nebenwirkungsfrei ist.

Es wurde insbesondere gefunden, daß die mit der aus Zellkultur gewonnenen, gereinigten Influenzavirus-Antigenpräparation, nicht nur alle für eine effektive Virus-Vakzine notwendigen Kriterien gemäß den CPMP-Richtlinien erfüllt, sondern daß das Antigen im Impfstoff im Vergleich zu einem konventionellen Impfstoff aus Eiern auch eine wesentlich höhere Immunogenität aufweist und zudem die für mögliche allergische Reaktionen verantwortlichen Hühnereiweiß-Proteine nicht enthält.

Die Versuche zeigen auch, daß der Anstieg des HA-Titers bei Schimpansen, immunisiert mit Influenzavirusantigen, isoliert aus Zellkultur, nicht nur wesentlich höher war als bei Tieren, die mit konventionellem Impfstoff bei gleicher Antigendosis immunisiert wurden, sondern auch, daß die Immunantwort bei Immunisierung mit einem Impfstoff mit einer geringeren Antigenmenge (1-5 µg Antigen/Dosis) und Adjuvans der HA-Titer nach 90 Tagen teilweise ein Drittel bis fast doppelt so hoch war wie bei der höheren Antigenmenge ohne Adjuvans (Tabelle 4).

Gemäß einem besonderen Aspekt der Erfindung enthält die erfindungsgemäße Vakzine vorzugsweise HA-Antigen, hergestellt und isoliert aus einer Influenzavirus-infizierten Zellkultur. Die Zellkultur kann dabei eine in serum- oder proteinfreiem Medium gezüchtete Vero-Zellkultur sein, wobei HA-Antigen vorzugsweise gemäß dem in WO 96/15231 beschriebenen Verfahren gewonnen wird. Eine derart erhaltene Influenzavirushaltige Lösung wird nach erfolgter Reinigung mittels kontinuierlicher Dichtezentrifugation im Gradienten und DNAse-Behandlung als gereinigte, konzentrierte Virusantigen-Präparation erhalten. Diese konzentrierte Präparation kann für die weitere Herstellung der erfindungsgemäßen Vakzine eingesetzt werden.

Ein weiterer Aspekt der Erfindung umfaßt ein Verfahren zur Herstellung einer Influenzavirus-Vakzine für höhere Säuger, insbesondere für Menschen, umfassend die Schritte
- Infizieren einer Zellkultur mit Influenzavirus
- Kultivieren der infizierten Zellen
- Ernten der produzierten Viren
- Reinigen der Viruspräparation
- Herstellen einer konzentrierten Viruspräparation
- Verdünnen der Viruspräparation auf einen HA-Antigengehalt zwischen 1 µg bis 5 µg Antigen pro Dosis und Zugabe von Aluminium als Adjuvans in einer Konzentration zwischen 0,05% bis 0,5%.

Die Herstellung des Influenzavirusantigens für die Vakzine erfolgt dabei vorzugsweise in einer serum- oder proteinfrei gezüchteten VERO-Zellkultur wie gemäß WO 96/15231 beschrieben. Die mit Influenzavirus-infizierten Zellen werden bis zum gewünschten Virustiter kultiviert und die Viren aus dem Kulturüberstand isoliert. Besonders wichtig ist dabei die Reinigung der gewonnenen Viruspräparation. Hierbei hat sich insbesondere ein Reinigungsverfahren umfassend die Schritte eines Saccharose-Gradienten, DNAse-Behandlung und Dia- und Sterilfiltration als geeignet gezeigt. Die derart gereinigte Präparation induziert auch nach Verdünnen auf 1/10 des in einer konventionellen Impfstoffdosis verabreichten HA-Antigengehalts und Adjuvantieren mit Aluminium eine noch höhere Immunantwort als die nicht-adjuvantierte Vakzinpräparation mit einem normalerweise als effektiv erachteten HA-Antigengehalt von 15 µg Antigen/Dosis.

Ein weiterer Aspekt der vorliegenden Erfindung umfaßt die Verwendung einer Influenzavirus Antigen Präparation mit einem HA-Antigengehalt von maximal zwischen 1 µg und 5 µg Antigen/Dosis und Aluminium als Adjuvans zur Herstellung eines Arzneimittels zur Prophylaxe oder Verhinderung von Influenzavirus-Infektionen.

Besonders bevorzugt ist dabei die erfindungsgemäße Verwendung zur Verhinderung einer Influenzavirus-Infektion bei Menschen. Da durch die Abwesenheit von Ei-spezifischen Proteinen auch die Wahrscheinlichkeit einer allergischen Reaktion gegen diese Proteine ausgeschlossen ist, ist der erfindungsgemäße Impfstoff insbesondere für die Anwendung bei Personengruppen die zu einer Hochrisiko-Gruppe gehören, wie etwa Asthmatiker, Allergiker, aber auch bei Menschen mit Immunsuppression und bei älteren Menschen, geeignet.

Aufgrund der im Rahmen der vorliegenden Erfindung durchgeführten Versuche an Schimpansen wurde festgestellt, daß insbesondere aus einer Zellkultur gewonnenes Influenzavirus-Antigen, adjuvantiert mit Aluminium, eine verbesserte Immunantwort auslöst und daher ein besseres Immunogen darstellt als ein durch konventionelle Methoden hergestelltes Antigen. Zudem bietet der erfindungsgemäße Impfstoff den Vorteil, daß er in einer Hühnereiweiß-Protein-freien Form vorliegt und daher die damit verbundenen Nebenwirkungen nicht aufweist.

Die Erfindung wird anhand der nachfolgenden Beispiele näher erläutert, ohne jedoch auf die Beispiele beschränkt zu sein.
Es zeigen:
**Tabelle 1:** HA-Titer von gepoolten Mausseren nach Vakzinierung mit Influenza A- oder B-Virus-Präparationen, gewonnen aus infizierten Zellkulturen oder bebrüteten Eiern.
**Tabelle 2:** Serokonversion von Schimpansen nach Immunisierung mit verschiedenen Influenzavirus-Vakzinpräparationen.
**Tabelle 3:** Geometrisches Mittel des HA-Titers (GMT) von Schimpansen nach Immunisierung mit verschiedenen Influenzavirus-Vakzinpräparationen.
**Tabelle 4:** Anstieg des GMT bei Schimpansen nach Immunisierung mit verschiedenen Influenzavirus-Vakzinpräparationen.
**Tabelle 5:** Protektiver Titer von Schimpansen nach Immunisierung mit verschiedenen Influenzavirus-Vakzinpräparationen.

### Beispiele:

### Beispiel 1:

### Herstellen einer Influenzavirus-Impfstoffpräparation

Influenzavirus wurde aus proteinfreier Vero-Zellkultur, infiziert mit Influenza A- oder B-Virusstamm, gemäß WO 96/15231 oder aus Allantoisflüssigkeit von infizierten bebrüteten Hühnereiern gewonnen.

Zur Herstellung einer Influenzaviruspräparation aus Zellkultur wurde der Überstand einer infizierten Vero-Zellkultur mit Formalin (Endkonzentration 0,025%) versetzt und die Viren bei 32°C für 24 Stunden inaktiviert. Dieses Material wurde durch zonale Zentrifugation in einem kontinuierlichen 0-50% Saccharose-Gradienten, DNAse-Behandlung, Diafiltration und Sterilfiltration gereinigt. Das gereinigte Material wurde bei -70°C gelagert. Das Endprodukt wurde auf Restkontaminationen getestet und pro Dosis wurden folgende Kriterien festgelegt:

| | |
|---|---|
| Hämagglutiningehalt: | ≥ 15 µg HA pro Stamm |
| Proteingehalt: | ≤ 250 µg |
| Saccharosegehalt: | ≤ 200 mg |
| Formalingehalt: | ≤ 5 µg |
| Benzonasegehalt: | ≤ 5 ng |
| Rest-DNA (VERO): | ≤ 100 pg |
| Endotoxingehalt: | ≤ 100 EU |
| Pyrogen: | frei |

### Beispiel 2 :

### Einfluß von Al(OH)₃ auf die Immunogenität von verschiedenen Influenzavirus-Impfstoff-Präparationen

Die isolierten Antigen-Präparationen aus Beispiel 1 wurden in PBS bis zu einem HA-Antigengehalt von 15 µg/ml verdünnt und gegebenenfalls mit Al(OH)₃ bis zu einer Endkonzentration von 0,2% adjuvantiert. Zur Herstellung einer Impfstoffpräparation von 1,5 µg wurde die Lösung entsprechend mit PBS enthaltend Al(OH)₃ verdünnt.

Jeweils 10 Mäuse wurden mit 1 ml der entsprechenden Präparation von 15 µg bzw. 1,5 µg Influenzavirus-HA-Antigen, jeweils mit und ohne Al(OH)₃ als Adjuvans immunisiert. Nach 4 Wochen wurde den Mäusen Blut entnommen und anschließend die Mäuse geboostert. 2 Wochen nach dem Booster wurden die Tiere entblutet. Das erhaltene Serum wurde im Influenza A- bzw. B-Virus-HämagglutinationsTest (HAI-Titer) nach der Methode von Palmer et al. (1977, Advanced laboratory technicals for immunological diagnostic, U.S. Dept. Health. Ed. Welfare. P.H.S. Atlanta, Immunology Ser. No. 2, A Procedural Guide to the Performance of Rubella Hemagglutination-Inhibition Tests, p. 3-64) getestet.

In Tabelle 1 sind die Ergebnisse des Experiments mit der Influenza A Virus-Stamm Johannesburg (A(H3N2)-Präparation und Influenza B Virus-Stamm B/Harbin-Präparation aus Vero-Zellkultur und NIB 34 (HG Johannesburg) aus Allantoisflüssigkeit zusammengefaßt. Das Serum der einzelnen Mäuse der verschiedenen Gruppen zu den Zeitpunkten nach 4 und 6 Wochen wurde gepoolt und der Antikörper-Titer mittels HAI-Test bestimmt.

Die Adjuvantierung mit Al(OH)₃ führte bereits nach 4 Wochen sowohl bei der geringeren als auch bei der höheren Antigendosis zu einem höheren Titeranstieg. Die geringere Dosis von 1,5 µg HA-Antigen adjuvantiert mit Al(OH)₃ ergab dabei sogar einen gleich hohen Titer wie die höhere Dosis; enthaltend 15 µg Antigen.

6 Wochen nach Immunisierung zeigten die Mäuse, die mit einer aus Zellkultur gewonnenen Impfstoffpräparation mit der höheren Antigendosis von 15 µg geimpft worden waren, mit und ohne Adjuvans den gleichen Titer. Die Präparation enthaltend eine 10-fach geringere Dosis von 1,5 µg und Al(OH)₃ führte nicht nur im Vergleich zum nicht-adjuvantierten Antigen, sondern auch gegenüber der adjuvantierten höheren Antigendosis zu einem wesentlich höheren HA-Titer. In der Präparation, enthaltend die höhere Dosis von Antigen, isoliert aus Zellkultur, hatte Aluminium keinen immunverstärkenden Effekt. Bei der Präparation isoliert aus Allantoisflüssigkeit hatte Al(OH)₃ nur einen geringen Einfluß auf die Immunantwort 6 Wochen nach Immunisierung, wogegen ein Titeranstieg bei der adjuvantierten Vakzine mit geringer Antigendosis festgestellt wurde.

Die Daten zeigen damit in Übereinstimmung mit den Ergebnissen von Davenport et al. (1968, J. Immunol. 100: 1139-1140), daß Aluminium einen immunverstärkenden Effekt bei Mäusen auf Influenza-Antigen aus Allantois hat.

Es war jedoch nicht zu erwarten, daß durch Herabsetzen der Antigenkonzentration auf 1/10 der "normalen" Dosis und Zugabe von Al(OH)₃ die Immunantwort gleich hoch der mit der 10-fachen Dosis ist bzw., daß der Titer nach Immunisierung mit aus Zellkultur gewonnenem Antigen sogar noch über den der höheren Antigendosis mit Al(OH)₃ ansteigt.

**Tabelle 1: HA-Titer von gepoolten Mausseren nach Vakzinierung mit Influenza A- oder B-Virus-Präparationen gewonnen aus Zellkultur oder bebrüteten Eiern**

| Gruppe | Antigen | Herkunft | Dosis (µg) | Adjuvans , | HAI-Titer | |
|---|---|---|---|---|---|---|
| | | | | | 4 Wochen | 6 Wochen |
| A | A/Johannesburg | VERO | 15 | | 320 | 1280 |
| B | A/Johannesburg | VERO | 1,5 | | 160 | 320 |
| C | A/Johannesburg | VERO | 15 | Al(OH)₃ | 640 | 1280 |
| D | A/Johannesburg | VERO | 1,5 | Al(OH)₃ | 640 | 2560 |
| | | | | | | |
| E | B/Harbin | VERO | 15 | | n.b.* | 320 |
| F | B/Harbin | VERO | 1,5 | | n.b. | 160 |
| G | B/Harbin | VERO | 15 | Al(OH)₃ | n.b. | 320 |
| K | B/Harbin | VERO | 1,5 | Al(OH)₃ | n.b. | 640 |
| | | | | | | |
| I | NIB 34 | Allantois | 15 | | 160 | 640 |
| J | NIB 34 | Allantois | 1,5 | | 160 | 320 |
| K | NIB 34 | Allantois | 15 | Al(OH)₃ | 640 | 1280 |
| L | NIB 34 | Allantois | 1,5 | Al(OH)₃ | 640 | 1280 |

| | | | | | | |
|---|---|---|---|---|---|---|
| * n.b. nicht bestimmt | | | | | | |

### Beispiel 3 :

### Untersuchungen zur Langzeit-Immunität von Schimpansen nach Verabreichung von Influenzavirus-Vakzine

Die Ergebnisse aus Beispiel 2 zeigen einen dramatischen Anstieg der Immunantwort bei Mäusen nach Immunisierung mit einer Vakzinpräparation mit einem reduzierten Antigengehalt enthaltend Al(OH)₃ als Adjuvans. Daher wurden weitere Untersuchungen durchgeführt, ob dieser Effekt auch bei höheren Säugern zu finden ist. Dazu wurde eine Langzeit-Studie bei Schimpansen durchgeführt.

Die Studie zur Langzeit-Immunität von Schimpansen nach Verabreichung einer Influenza-Ganzvirusvakzine wurde an 4 Gruppen von insgesamt 44 Schimpansen durchgeführt. Dazu wurde jede Gruppe folgendermaßen immunisiert:
Gruppe I: 13 Schimpansen wurden mit einer Influenza-Vakzinpräparation aus einer Vero-Zellkultur mit jeweils 15 µg pro Stamm immunisiert.
Gruppe II: 5 Schimpansen wurden mit einer Influenza-Vakzinpräparation aus einer Vero-Zellkultur mit jeweils 5 µg pro Stamm und Al(OH)₃ als Adjuvans immunisiert.
Gruppe III: 13 Schimpansen wurden mit einer Influenza-Vakzinpräparation aus einer Vero-Zellkultur mit jeweils 1,5 µg pro Stamm und Al(OH)₃ als Adjuvans immunisiert.
Gruppe IV: 13 Schimpansen wurden mit einer aus Allantoisflüssigkeit von bebrüteten Eiern gewonnenen Influenzavirus-Präparation mit 15 µg pro Stamm immunisiert.

Blutproben wurden von den 44 Schimpansen nach 0, 10, 30 und 90 Tagen nach Vakzinierung entnommen. Nach einer RDE (receptor destroying enzyme)-Behandlung wurden die Schimpansen-Seren für 45 Minuten bei 56°C inaktiviert und auf anti-Hämagglutinations-Antikörper gegen Influenzavirus-Wildtypstamm Texas 36(A(H1N1), Nanchang (A/H3N2) und B/Harbin (B) mittels Hämagglutinationstest getestet. Die Interpretation erfolgte über die CPMP-Richtlinien (Committee for Propietary Medicinal products (CPMP): Note for Guidance on Harmonisation of Requirements for Influenza Vaccines (CPMP/BWP/214/96), 17. July 1996: 20-21). Entsprechend den Richtlinien muß eine effektive Vakzine mindestens eines der folgenden Kriterien erfüllen:

Prozent der Serokonversion oder ein signifikanter Anstieg des anti-Hämagglutinationstiters (≥ 40) (Tabelle 2) > 40%.

Anstieg des GMT (geometrischen Mittel des Titers) um > 2,5 (Tabelle 3 und 4).
der Anteil von Subjekten, die einen HAI-Titer von ≥ 40 (Protektionstiter) erreichen, sollte > 70% sein (Tabelle 5).

Die Ergebnisse sind in den Tabellen 2 bis 5 zusammengefaßt und zeigen, daß ebenso wie die konventionelle Vakzine aus Allantoisflüssigkeit die aus Vero-Zellkultur gewonnene InfluenzavirusPräparation alle 3 Kriterien der CPMP-Richtlinien erfüllt. Die aus Vero-Zellkultur gewonnene Impfstoffpräparation ist daher nicht nur genauso immunogen wie die aus Allantoisflüssigkeit, sondern zeigt aufgrund der Daten auch eine höhere Immunogenität.

Zudem zeigte sich überraschenderweise, daß eine aus Vero-Zellkultur gewonnene Präparation, die 1 : 3 (5 µg pro Stamm) oder 1 : 10 (1,5 µg pro Stamm) verdünnt und mit Al(OH)₃ adjuvantiert ist, ebenfalls alle 3 Kriterien ab Tag 30 und 90 nach Immunisierung erfüllt.

So zeigt insbesondere Tabelle 2, daß 30 Tage nach Immunisierung die Schimpansen, die mit einer gereinigten Influenzavirus-Vakzinpräparation aus Zellkultur geimpft wurden, eine höhere Serokonversion im Vergleich zu denen, die mit konventionell hergestelltem Impfstoff aus Allantois geimpft wurden, aufweisen. Besonders überraschend war zudem, daß bei der Präparation mit einer geringeren Antigendosis (5 µg bzw. 1,5 µg), enthaltend Al(OH)₃ als Adjuvans, die Serokonversion am höchsten war.

Die Tendenz, daß die Immunantwort bei Schimpansen, immunisiert mit einer Vakzinpräparation mit einer geringeren Antigendosis und einer Aluminiumverbindung als Adjuvans, höher ist als bei den Tieren, die eine "normal" hohe Antigendosis ohne Adjuvans erhalten haben, ist auch über den Anstieg des HA-Titers festzustellen (Tabelle 3 und 4). Dabei ist eindeutig ein.doppelt so hoher Anstieg des HA-Titers nach 30 Tagen bei Influenzavirus-Antigen, isoliert aus Zellkultur, im Vergleich zu Antigen aus Allantoisflüssigkeit nachzuweisen, wobei dieser Effekt bei geringer Antigendosis plus Adjuvans noch wesentlich deutlicher ausgeprägt ist (Tabelle 4). 90 Tage nach Immunisierung war der Anstieg des GMT mit der aus Zellkultur gewonnenen, gereinigten Impfstoffpräparation fast doppelt so hoch wie der des konventionellen Impfstoffes.

Vergleiche der Immunogenität von Influenzavirusantigen-Präparationen, isoliert aus Allantoisflüssigkeit von bebrüteten Eiern, oder aus einer Vero-Zellkultur zeigten, daß das aus Zellkultur gewonnene Antigen eine 2- bis 4-fach höhere Immunogenität als das aus Allantoisflüssigkeit aufweist.

**Tabelle 2: Serokonversion von Schimpansen nach Immunisierung mit verschiedenen Influenzavirus-Vakzinpräparationen**

| | % mit 4 fach oder > HI-Titer Anstieg Stamm | | | |
|---|---|---|---|---|
| | Tag | Texas 36 | Nanchang | B/Harbin |
| Gruppe I | | | | |
| Vero-Zellmaterial | 10 | 69% (9/13) | 69% (9/13) | 62% (8/13) |
| 15 µg/Stamm | 30 | 85% (11/13) | 77% (10/13) | 85%(11/13) |
| | 90 | 69% (9/13) | 69% (9/13) | 85% (11/13) |
| | | | | |
| Gruppe II | | | | |
| Vero-Zellmaterial | 10 | 60% ( /5) | 60% (3/5) | 60% ( 3/5) |
| 5 µg/Stamm (Al(OH)₃) | 30 | 100% (5/5) | 80% (4/5) | 100% (5/5) |
| | 90 | 80% (4/5) | 80% (4/5) | 100% (5/5) |
| | | | | |
| Gruppe III | | | | |
| Vero-Zellmaterial | 10 | 54% ( 7/13) | 69% ( 9/13) | 46% ( 6/13) |
| 1.5µg/Stamm (A1(OH)₃) | 30 | 92% (12/13) | 92% (12/13) | 85% (11/13) |
| | 90 | 85% (11/13) | 85% (11/13) | 77% (10/13) |
| | | | | |
| Gruppe IV | | | | |
| Antigen aus Eiern | 10 | 46% (6/13) | 54% (7/13) | 62% ( 8/13) |
| 15 µg/Stamm | 30 | 69% (9/13) | 69% (9/13) | 77% (10/13) |
| | 90 | 67% (8/12) | 67% (8/12) | 83% (10/12) |
| | | | | |
| CPMP-Kriterien für Effizienz | | > 40% | > 40% | > 40% |

**Tabelle 3: Geometrisches Mittel des HA-Titers (GMT) von Schimpansen nach Immunisierung mit verschiedenen Influenzavirus-Vakzinpräparationen**

| | GMT (Geometrisches Mittel - Titer) Stamm | | | |
|---|---|---|---|---|
| | Tag | Texas 36 | Nanchang | B/Harbin |
| Gruppe I-IV | 0 | 21,6 | 11,0 | 7,5 |
| Gruppe I | | | | |
| | 0 | 21,1 | 10,0 | 7,3 |
| Vero-Zellmaterial | 10 | 80,0 | 36,0 | 22,3 |
| 15 µg/Stamm | 30 | 168,8 | 84,0 | 58,1 |
| | 90 | 52,2 | 49,5 | 42,2 |
| Gruppe II | | | | |
| | 0 | 17,4 | 10,0 | 6,6 |
| Vero-Zelimaterial | 10 | 52,8 | 30,3 | 13,2 |
| 5 µg/ Stamm (Al(OH)₃) | 30 | 160,0 | 69,6 | 46,0 |
| | 90 | 69,6 | 60,6 | 46,0 |
| Gruppe III | | | | |
| | 0 | 18,0 | 10,6 | 7,3 |
| Vero-Zellmaterial | 10 | 40,0 | 44,5 | 14,1 |
| 1,5 µg/ Stamm (Al(OH)₃) | 30 | 208,9 | 104,4 | 64,6 |
| | 90 | 80,0 | 71,9 | 55,1 |
| Gruppe IV | | | | |
| | 0 | 29,1 | 13,1 | 8,5 |
| Antigen aus Eiern | 10 | 64,6 | 34,1 | 22,3 |
| 15 µg/Stamm | 30 | 160,0 | 64,6 | 58,1 |
| | 90 | 84,8 | 59,9 | 44,3 |

**Tabelle 4: Anstieg des GMT bei Schimpansen nach Immunisierung mit verschiedenen Influenzavirus-Vakzinpräparationen**

| | GMT -Anstieg (vor/nach-Vakzinierung) Stamm | | | |
|---|---|---|---|---|
| | Tag | Texas 36 | Nanchang | B/Harbin |
| Gruppe I | | | | |
| Vero-Zeilmaterial | 10 | 3,8 | 3,6 | 3,1 |
| 15 µg/ Stamm | 30 | 8,0 | 8,4 | 8,0 |
| | 90 | 2,5 | 5,0 | 5,9 |
| | | | | |
| Gruppe II | | | | |
| Vero-Zellmaterial | 10 | 3,0 | 3,0 | 2,0 |
| 5 µg/Stamm (Al(OH)₃) | 30 | 9,2 | 7,0 | 7,0 |
| | 90 | 4,0 | 6,1 | 7,0 |
| | | | | |
| Gruppe III | | | | |
| Vero-Zellmaterial | 10 | 2,2 | 4,2 | 1,9 |
| 1,5 µg/ Stamm (Al(OH)₃) | 30 | 11,6 | 9,9 | 8,9 |
| | 90 | 4,4 | 6,8 | 7,6 |
| | | | | |
| Gruppe IV | | | | |
| Antigen aus Eiern | 10 | 2,2 | 2,6 | 2,6 |
| 15 µg/ Stamm | 30 | 5,5 | 4,9 | 6,8 |
| | 90 | 2,9 | 4,6 | 5,1 |
| | | | | |
| CPMP Kriterien für Effizienz | | > 2,5 | > 2,5 | > 2,5 |

**Tabelle 5: Protektiver Titer von Schimpansen nach Immunisierung mit verschiedenen Influenzavirus-Vakzinpräparationen**

| | | % mit HI-Titer > 40 Stamm | | |
|---|---|---|---|---|
| | Tag | Texas 36 | Nanchang | B/Harbin |
| Gruppe I-IV | 0 | 25% (11/44) | 21% ( 9/44) | 14% (6/44) |
| Gruppe I | | | | |
| | 0 | 23% ( 3/13) | 15% ( 2/13) | 15% ( 2/13) |
| Vero-Zellmaterial | 10 | 92% (12/13) | 85% (11/13) | 39% ( 5/13) |
| 15 µg/ Stamm | 30 | 100% (13/13) | 92% (12/13) | 100% (13/13) |
| | 90 | 92% (12/13) | 85% (11/13) | 85% (11/13) |
| Gruppe II | | | | |
| | 0 | 20% (1/5) | 20% (1/5) | 0% (0/5) |
| Vero-Zellmaterial | 10 | 80% (4/5) | 80% (4/5) | 20% (1/5) |
| 5 µg/ Stamm (Al(OH)₃) | 30 | 100% (5/5) | 100% (5/5) | 100%(5/5) |
| | 90 | 100% (5/5) | 100% (5/5) | 100% (5/5) |
| Gruppe III | | | | |
| | 0 | 15% ( 2/13) | 15% ( 2/13) | 15% ( 2/13) |
| Vero-Zellmaterial | 10 | 69% ( 9/13) | 69% ( 9/13) | 23% ( 3/13) |
| 1,5 µg/ Stamm (Al(OH)₃) | 30 | 100% (13/13) | 100% (13/13) | 100% (13/13) |
| | 90 | 100% (13/13) | 100% (13/13) | 92% (2/13) |
| Gruppe IV | | | | |
| | 0 | 39% ( 5/13) | 31% ( 4/13) | 15% ( 2/13) |
| Antigen aus Eiern | 10 | 85% (11/13) | 77% (10/13) | 46% ( 6/13) |
| 15 µg/ Stamm | 30 | 100% (13/13) | 100% (13/13) | 92%(12/13) |
| | 90 | 100% (12/12) | 83% ( 10/12 | 92% (11/12) |
| CPMP Kriterien für Effizienz | | > 70% | > 70% | > 70% |

## Patentansprüche

1. Influenzavirus-Vakzine, enthaltend eine aus Zellkultur gewonnene Influenzavirus-Antigen Präparation mit einem HA-Antigengehalt zwischen 1 µg und 5 µg/Dosis und Aluminium als Adjuvans.

2. Influenzavirus-Vakzine nach Anspruch 1, **dadurch gekennzeichnet, daß** sie einen HA-Antigengehalt von 1 µg bis 2,5 µg/Dosis, vorzugsweise von 1,5 µg/Dosis aufweist.

3. Influenzavirus-Vakzine nach Anspruch 1 oder 2, **dadurch gekennzeichnet, daß** sie Aluminium als Aluminiumhydroxid oder Aluminiumphosphat enthält.

4. Influenzavirus-Vakzine nach einem der Ansprüche 1 bis 3, **dadurch gekennzeichnet, daß** sie Aluminium mit einer Endkonzentration von 0,05% bis 0,5% enthält.

5. Influenzavirus-Vakzine nach einem der Ansprüche 1 bis 4, **dadurch gekennzeichnet, daß** sie gereinigte Influenzaviren enthält.

6. Influenzavirus-Vakzine nach einem der Ansprüche 1 bis 5, **dadurch gekennzeichnet, daß** sie zur Anwendung als Vakzine bei Menschen geeignet ist.

7. Verfahren zur Herstellung einer Influenzavirus-Vakzine umfassend die Schritte
- Infizieren einer Zellkultur mit Influenzavirus
- Kultivieren der infizierten Zellen
- Ernten der produzierten Viren
- Reinigen der Viruspräparation
- Herstellen einer konzentrierten Viruspräparation
- Verdünnen der Viruspräparation auf einen HA-Antigengehalt zwischen 1 µg bis 5 µg Antigen pro Dosis und Zugabe von Aluminium als Adjuvans in einer Endkonzentration zwischen 0,05% bis 0,5%.

8. Verwendung einer Influenzavirus-Antigenpräparation mit einem HA-Antigengehalt zwischen 1 µg und 5 µg Antigen/Dosis und Aluminium als Adjuvans zur Herstellung eines Arzneimittels zur Prophylaxe oder Verhinderung von Influenzavirus-Infektionen.

## Claims

1. Influenza virus vaccine comprising an influenza virus antigen preparation isolated from a cell culture and having an HA antigen content of between 1 µg and 5 µg/dose and aluminium as an adjuvant.

2. Influenza virus vaccine according to Claim 1, **characterized in that** it has an HA antigen content of 1 µg to 2.5 µg/dose, preferably of 1.5 µg/dose.

3. Influenza virus vaccine according to Claim 1 or 2, **characterized in that** it comprises aluminium as aluminium hydroxide or aluminium phosphate.

4. Influenza virus vaccine according to one of Claims 1 to 3, **characterized in that** it comprises aluminium in a final concentration of 0.05% to 0.5%.

5. Influenza virus vaccine according to one of Claims 1 to 4, **characterized in that** it comprises purified influenza viruses.

6. Influenza virus vaccine according to one of Claims 1 to 5, **characterized in that** it is suitable for use as a vaccine in humans.

7. Method for the preparation of an influenza virus vaccine, comprising the steps of
- infecting a cell culture with influenza virus
- cultivating the infected cells
- harvesting the viruses produced
- purifying the virus preparation
- producing a concentrated virus preparation
- diluting the virus preparation to an HA antigen content of between 1 µg and 5 µg of antigen per dose and adding aluminium as an adjuvant in a final concentration of between 0.05% and 0.5%.

8. The use of an influenza virus antigen preparation having an HA antigen content of between 1 µg and 5 µg of antigen/dose and aluminium as an adjuvant in the manufacture of a drug for the prophylaxis or prevention of influenza virus infections

## Revendications

1. Vaccin contre le virus de la grippe contenant une préparation d'antigène du virus de la grippe obtenue à partir d'une culture cellulaire, avec une teneur en antigène HA comprise entre 1 µg et 5 µg/dose et de l'aluminium comme adjuvant.

2. Vaccin contre le virus de la grippe selon la revendication 1, **caractérisé en ce qu'**il présente une teneur en antigène HA comprise entre 1 µg et 2,5 µg/dose, de préférence de 1,5 µg/dose.

3. Vaccin contre le virus de la grippe selon la revendication 1 ou 2, **caractérisé en ce qu'**il contient de l'aluminium sous la forme d'hydroxyde d'aluminium ou de phosphate d'aluminium.

4. Vaccin contre le virus de la grippe selon l'une quelconque des revendications 1 à 3, **caractérisé en ce qu'**il contient de l'aluminium en concentration finale de 0,05% à 0,5%.

5. Vaccin contre le virus de la grippe selon l'une quelconque des revendications 1 à 4, **caractérisé en ce qu'**il contient des virus de la grippe purifiés.

6. Vaccin contre le virus de la grippe selon l'une quelconque des revendications 1 à 5, **caractérisé en ce qu'**il convient à une utilisation comme vaccin chez les humains.

7. Procédé de fabrication d'un vaccin contre le virus de la grippe, comprenant les étapes suivantes :
- l'infection d'une culture cellulaire par le virus de la grippe,
- la culture des cellules infectées,
- la récolte des virus produits,
- la purification de la préparation virale,
- la fabrication d'une préparation virale concentrée et
- la dilution de la préparation virale jusqu'à une teneur en antigène HA comprise entre 1 µg et 5 µg d'antigène par dose, et l'addition d'aluminium comme adjuvant en concentration finale comprise entre 0,05% et 0,5%.

8. Utilisation d'une préparation d'antigène du virus de la grippe avec une teneur en antigène HA comprise entre 1 µg et 5 µg d'antigène/dose et de l'aluminium comme adjuvant pour fabriquer un produit pharmaceutique destiné à la prophylaxie et à la prévention d'infections par le virus de la grippe.
